# EUROPÄISCHE PATENTANMELDUNG

(11) **EP 1 291 422 A2**
(43) Veröffentlichungstag der Anmeldung: **12.03.2003**
(21) Anmeldenummer: 02090198.9
(22) Anmeldetag: 07.04.1999
(51) Int. Cl.: C12N 15/10, G01N 33/574, C07K 16/18, C12N 5/10, C12Q 1/68

(54) **Menschliche Nukleinsäuresequenzen aus Ovartumorgewebe**

(30) Priorität: 09.04.1998 DE 19817557
(62) Teilanmeldung aus: 99945687.4
(71) Anmelder: metaGen Pharmaceuticals GmbH, 13347 Berlin (DE)
(72) Erfinder: Specht, Thomas Dr., 14169 Berlin (DE); Hinzmann, Bernd Dr., 13127 Berlin (DE); Schmitt, Armin Dr., 96149 Breitengussbach (DE); Pilarsky, Christian Dr., 14532 Stahnsdorf (DE); Dahl, Edgar Dr., 14480 Potsdam (DE); Rosenthal, Andr- Prof. Dr., 10115 Berlin (DE)
(74) Vertreter: Jungblut, Bernhard Jakob, Dr.

(57) **Zusammenfassung**

Es werden menschliche Nukleinsäuresequenzen -mRNA, cDNA, genomische Sequenzen- aus Ovartumorgewebe, die für Genprodukte oder Teile davon kodieren, und deren Verwendung beschrieben

Es werden weiterhin die über die Sequenzen erhältlichen Polypeptide und deren Verwendung beschrieben.

## Beschreibung

Die Erfindung betrifft eine menschliche Nukleinsäuresequenz aus Ovartumorgewebe, die für Genprodukte oder Teile davon kodiert, deren funktionales Gen, das mindestens ein biologisch aktives Polypeptid kodiert und deren Verwendung.
Die Erfindung betrifft weiterhin die über die Sequenzen erhältlichen Polypeptide und deren Verwendung.

Eine der Hauptkrebstodesursachen bei Frauen ist das Ovarialkarzinom, für dessen Bekämpfung neue Therapien notwendig sind. Bisher verwendete Therapien, wie z.B. Chemotherapie, Hormontherapie oder chirugische Entfernung des Tumorgewebes, führen häufig nicht zu einer vollständigen Heilung.

Das Phänomen Krebs geht häufig einher mit der Über- oder Unterexpression gewisser Gene in den entarteten Zellen, wobei noch unklar ist, ob diese veränderten Expressionsraten Ursache oder Folge der malignen Transformation sind. Die Identifikation solcher Gene wäre ein wesentlicher Schritt für die Entwicklung neuer Therapien gegen Krebs. Der spontanen Entstehung von Krebs geht häufig eine Vielzahl von Mutationen voraus. Diese können verschiedenste Auswirkungen auf das Expressionsmuster in dem betroffenen Gewebe haben, wie z.B. Unter- oder Überexpression, aber auch Expression verkürzter Gene. Mehrere solcher Veränderungen durch solche Mutationskaskaden können schließlich zu bösartigen Entartungen führen. Die Komplexität solcher Zusammenhänge erschwert die experimentelle Herangehensweise sehr.

Für die Suche nach Kandidatengenen, d.h. Genen, die im Vergleich zum Tumorgewebe im normalen Gewebe stärker exprimiert werden, wird eine Datenbank verwendet, die aus sogenanten ESTs besteht. ESTs (Expressed Sequence Tags) sind Sequenzen von cDNAs, d.h. revers transkribierten mRNAs, den Molekülen also, die die Expression von Genen widerspiegeln. Die EST-Sequenzen werden für normale und entartete Gewebe ermittelt. Solche Datenbanken werden von verschiedenen Betreibern z.T. kommerziell angeboten. Die ESTs der LifeSeq-Datenbank, die hier verwendet wird, sind in der Regel zwischen 150 und 350 Nukleotide lang. Sie representieren ein für ein bestimmtes Gen unverkennbares Muster, obwohl dieses Gen normalerweise sehr viel länger ist ( > 2000 Nukleotide). Durch Vergleich der Expressionsmuster von normalen und Tumorgewebe können ESTs identifiziert werden, die für die Tumorentstehung und -proliferation wichtig sind. Es besteht jedoch folgendes Problem: Da durch unterschiedliche Konstruktionen der cDNA-Bibliotheken die gefundenen EST-Sequenzen zu unterschiedlichen Regionen eines unbekannten Gens gehören können, ergäbe sich in einem solchen Fall ein völlig falsches Verhältnis des Vorkommens dieser ESTs in dem jeweiligen Gewebe. Dieses würde erst bemerkt werden, wenn das vollständige Gen bekannt ist und somit die ESTs dem gleichen Gen zugeordnet werden können.

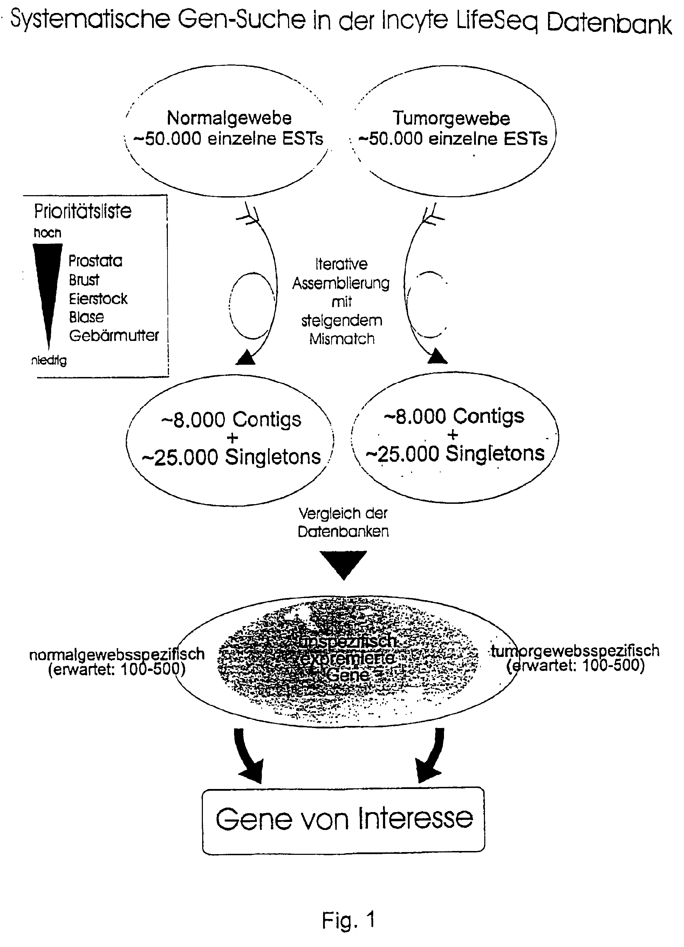

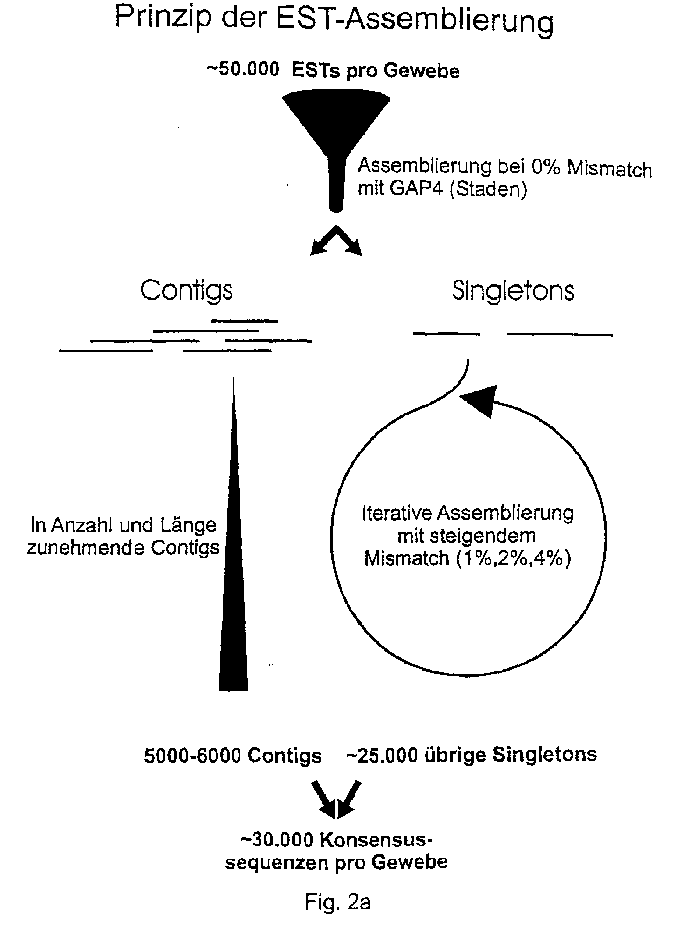

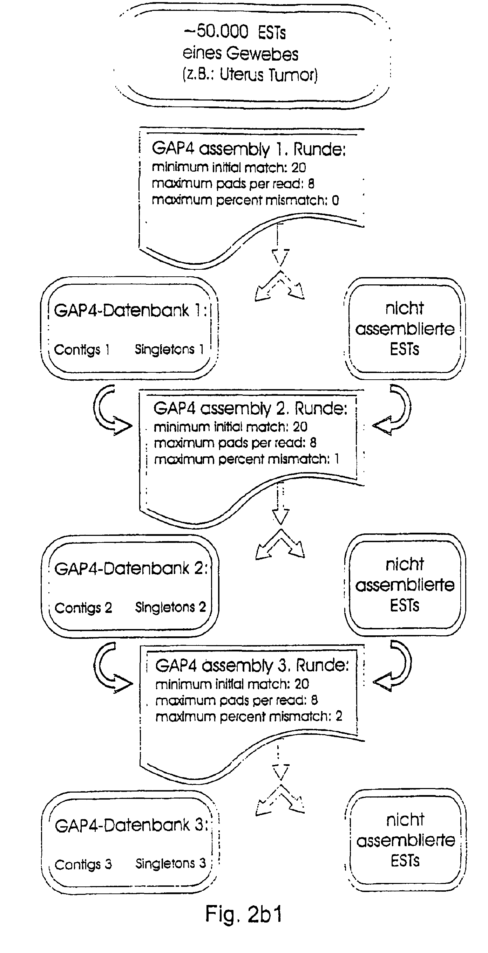

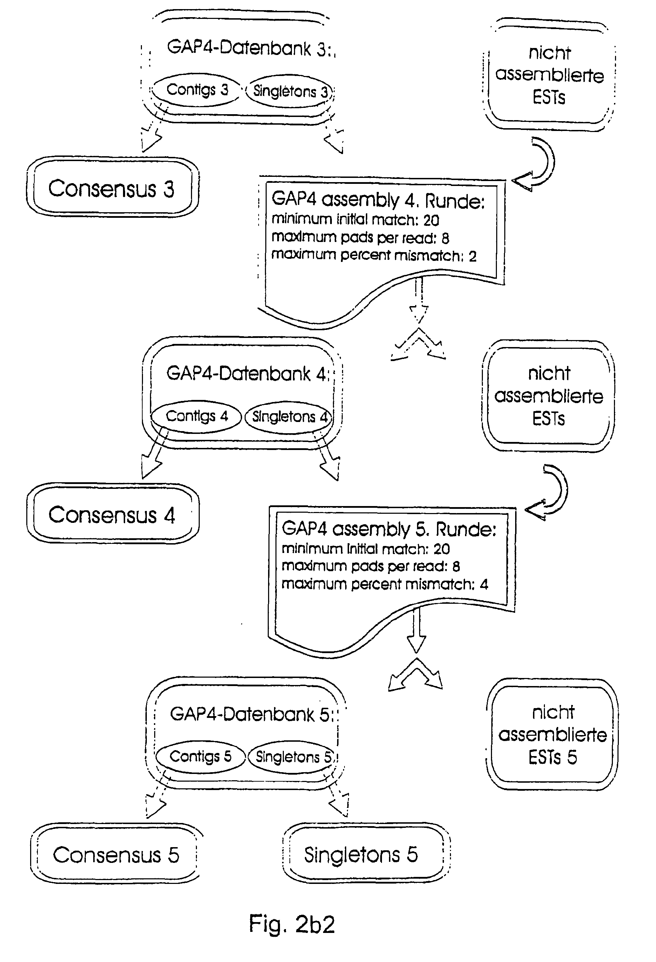

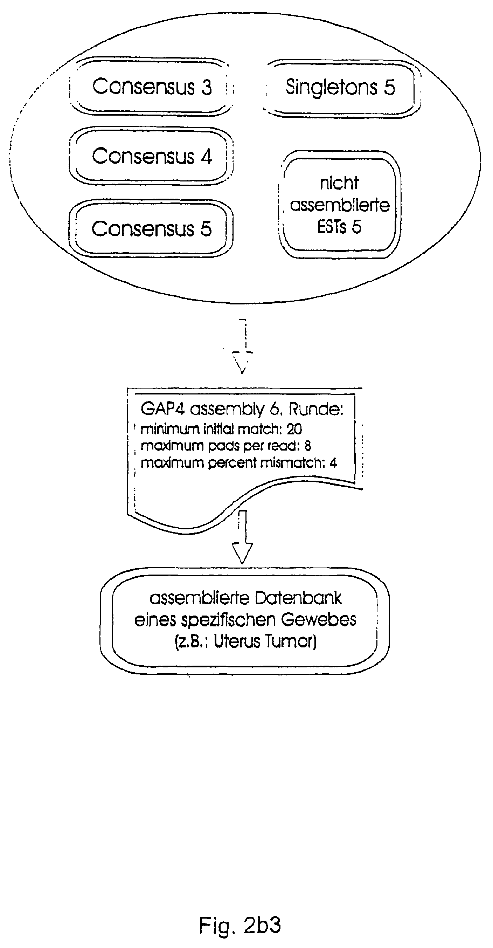

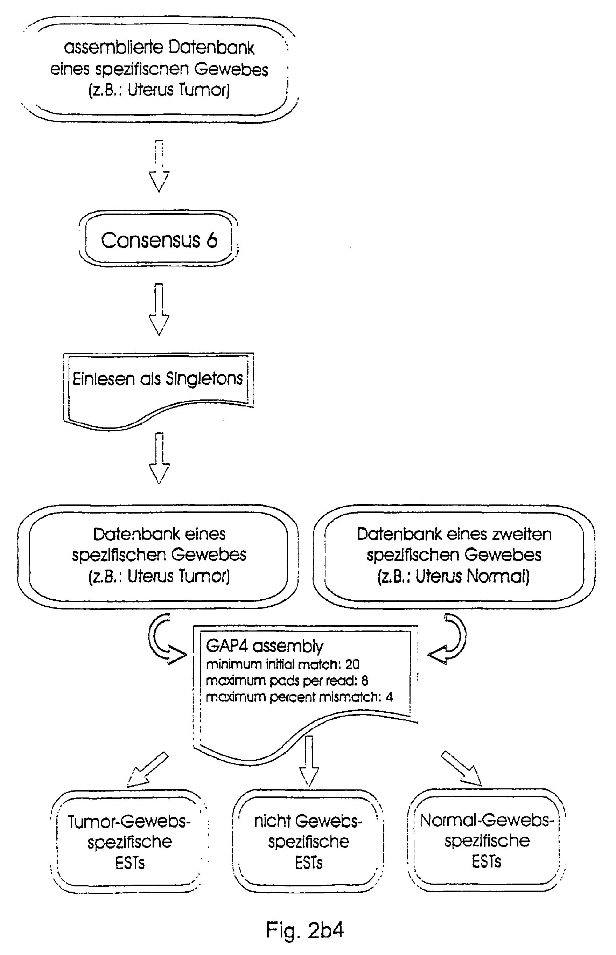

Es wurde nun gefunden, daß diese Fehlermöglichkeit verringert werden kann, wenn zuvor sämtliche ESTs aus dem jeweiligen Gewebstyp assembliert werden, bevor die Expressionsmuster miteinander verglichen werden. Es wurden also überlappende ESTs ein und desselben Gens zu längeren Sequenzen zusammengefaßt (s. Fig. 1, Fig. 2a und Fig.3). Durch diese Verlängerung und damit Abdeckung eines wesentlich größeren Genbereichs in jeder der jeweiligen Banken sollte der oben beschriebene Fehler weitgehenst vermieden werden. Da es hierzu keine bestehenden Softwareprodukte gab, wurden Programme für das Assemblieren von genomischen Abschnitten verwendet, die abgewandelt eingesetzt und durch eigene Programme ergänzt wurden. Ein Flowchart der Assemblierungsprozedur ist in Fig. 2b1 - 2b4 dargestellt.

Es konnte nun die Nukleinsäure-Sequenz Seq. ID No 115 gefunden werden, die als Kandidatengen beim Ovarialkarzinom eine Rolle spielen.

Die Erfindung betrifft somit Nukleinsäure-Sequenzen, die ein Genprodukt oder ein Teil davon kodieren, umfassend
a) eine Nukleinsäure-Sequenz Seq. ID No 115,
b) eine allelische Variation der unter a) genannten Nukleinsäure-Sequenz
oder
c) eine Nukleinsäure-Sequenz, die komplementär zu den unter a) oder b) genannten Nukleinsäure-Sequenzen ist.

Die Erfindung betrifft auch die Nukleinsäure-Sequenz Seq. ID No 115, die im Ovartumorgewebe erhöht exprimiert ist.

Die Erfindung betrifft ferner Nukleinsäure-Sequenzen, umfassend einen Teil der oben genannten Nukleinsäure-Sequenzen, in solch einer ausreichenden Größe, daß sie mit der Sequenz Seq. ID No 115 hybridisiert.

Die erfindungsgemäßen Nukleinsäure-Sequenzen weisen im allgemeinen eine Länge von mindestens 50 bis 4500 bp, vorzugsweise eine Länge von mindestens 150 bis 4000 bp, insbesondere eine Länge von 450 bis 3500 bp auf.

Mit der erfindungsgemäßen Teilsequenz Seq. ID No 115 können gemäß gängiger Verfahrenspraxis auch Expressionskassetten konstruiert werden, wobei auf der Kassette mindestens eine der erfindungsgemäßen Nukleinsäure-Sequenzen zusammen mit mindestens einer dem Fachmann allgemein bekannten Kontrolloder regulatorischen Sequenz, wie z. B. einem geeigneten Promotor, kombiniert wird. Die erfindungsgemäßen Sequenzen können in sense oder antisense Orientierung eingefügt sein.

In der Literatur sind ist eine große Anzahl von Expressionskassetten bzw. Vektoren und Promotoren bekannt, die verwendet werden können.

Unter Expressionskassetten bzw. Vektoren sind zu verstehen: 1. bakterielle, wie z. B., phagescript, pBs, φX174, pBluescript SK, pBs KS, pNH8a, pNH16a, pNH18a, pNH46a (Stratagene), pTrc99A, pKK223-3, pKK233-3, pDR540, pRIT5 (Pharmacia),
2. eukaryontische, wie z. B. pWLneo, pSV2cat, pOG44, pXT1, pSG (Stratagene), pSVK3, pBPV, pMSG, pSVL (Pharmacia).

Unter Kontroll- oder regulatorischer Sequenz sind geignete Promotoren zu verstehen. Hierbei sind zwei bevorzugte Vektoren der pKK232-8 und der PCM7 Vektor. Im einzelnen sind folgende Promotoren gemelnt: lacl, lacZ, T3, T7, gpt, lambda P_{R}, trc, CMV, HSV Thymidin-Kinase, SV40, LTRs aus Retrovirus und Maus Metallothionein-I.

Die auf der Expressionskassette befindlichen DNA-Sequenzen können ein Fusionsprotein kodieren, das ein bekanntes Protein und ein biologisch aktives Polypeptid-Fragment umfaßt.

Die Expressionskassetten sind ebenfalls Gegenstand der vorliegenden Erfindung.

Die erfindungsgemäßen Nukleinsäure-Fragmente können zur Herstellung von Vollängen-Genen verwendet werden. Die erhältlichen Gene sind ebenfalls Gegenstand der vorliegenden Erfindung.

Die Erfindung betrifft auch die Verwendung der erfindungsgemäßen Nukleinsäure-Sequenzen, sowie die aus der Verwendung erhältlichen Gen-Fragmente.

Die erfindungsgemäßen Nukleinsäure-Sequenzen können mit geeigneten Vektoren in Wirtszellen gebracht werden, in denen als heterologer Teil die auf den Nukleinsäure-Fragmenten enthaltene genetischen Information befindet, die exprimiert wird.

Die die Nukleinsäure-Fragmente enthaltenden Wirtszellen sind ebenfalls Gegenstand der vorliegenden Erfindung.

Geeignete Wirtszellen sind z. B. prokaryontische Zellsysteme wie E. coli oder eukaryontische Zellsysteme wie tierische oder humane Zellen oder Hefen.

Die erfindungsgemäßen Nukleinsäure-Sequenzen können in sense oder antisense Form verwendet werden.

Die Herstellung der Polypeptide oder deren Fragment erfolgt durch Kultivierung der Wirtszellen gemäß gängiger Kultivierungsmethoden und anschließender Isolierung und Aufreinigung der Peptide bzw. Fragmente, ebenfalls mittels gängiger Verfahren.
Die Erfindung betrifft ferner Nukleinsäure-Sequenzen, die mindestens eine Teilsequenz eines biologisch aktiven Polypeptids kodieren.

Die Erfindung betrifft ferner die Polypeptid-Sequenzen, die mindestens eine 80%ige Homologie, insbesondere eine 90%ige Homologie zu den erfindungsgemäßen Polypeptid-Teilsequenzen aufweisen.

Die Erfindung betrifft auch Antikörper, die gegen ein Polypeptid oder Fragment davon gerichtete sind, welche von der erfindungsgemäßen Nukleinsäure der Sequenz Seq. ID No 115 kodiert wird.

Unter Antikörper sind insbesondere monoklonale Antikörper zu verstehen.

Die erfindungsgemäßen Antikörper können u.a. durch ein Phage Display Verfahren identifiziert werden. Auch diese Antikörper sind Gegenstand der Erfindung.

Die erfindungsgemäßen Polypeptid-Teilsequenzen können in einem Phage Display Verfahren verwendet werden. Die mit diesem Verfahren identifizierten Polypeptide, die an die erfindungsgemäßen Polypeptid-Teilsequenzen binden, sind auch Gegenstand der Erfindung.

Ebenso können die erfindungsgemäßen Nukleinsäure-Sequenzen in einem Phage Display Verfahren verwendet werden.

Die Erfindung betrifft auch Phage-Display Phagen, die gegen ein Polypeptid oder ein Fragment gerichtet sind, welche von der erfindungsgemäßen Nukleinsäure der Sequenz Seq. ID No 115 kodiert werden.

Die erfindungsgemäßen Polypeptide können auch als Tool zum Auffinden von Wirkstoffen gegen das Ovarialkarzinom verwendet werden, was ebenfalls Gegenstand der vorliegenden Erfindung ist.

Ebenfalls Gegenstand der vorliegenden Erfindung ist die Verwendung der Nukleinsäure-Sequenzen gemäß der Sequenz Seq. ID No 115 zur Expression von Polypeptiden, die als Tools zum Auffinden von Wirkstoffen gegen das Ovarialkarzinom verwendet werden können.

Die Erfindung betrifft auch die Verwendung der gefundenen Polypeptid-Teilsequenzen als Arzneimittel in der Gentherapie zur Behandlung gegen das Ovarialkarzinom, bzw. zur Herstellung eines Arzneimittels zur Behandlung gegen das Ovarialkarzinom.

Die Erfindung betrifft auch Arzneimittel, die mindestens eine Polypeptid-Teilsequenz enthalten.

Die gefundenen erfindungsgemäßen Nukleinsäure-Sequenzen können auch genomische oder mRNA-Sequenzen sein.

Die Erfindung betrifft auch genomische Gene, ihre Exon- und Intronstruktur und deren Spleißvarianten, erhältlich aus der cDNA der Sequenz Seq. ID No 115, sowie deren Verwendung zusammen mit geeigneten regulativen Elementen, wie geeigneten Promotoren und/ oder Enhancern.

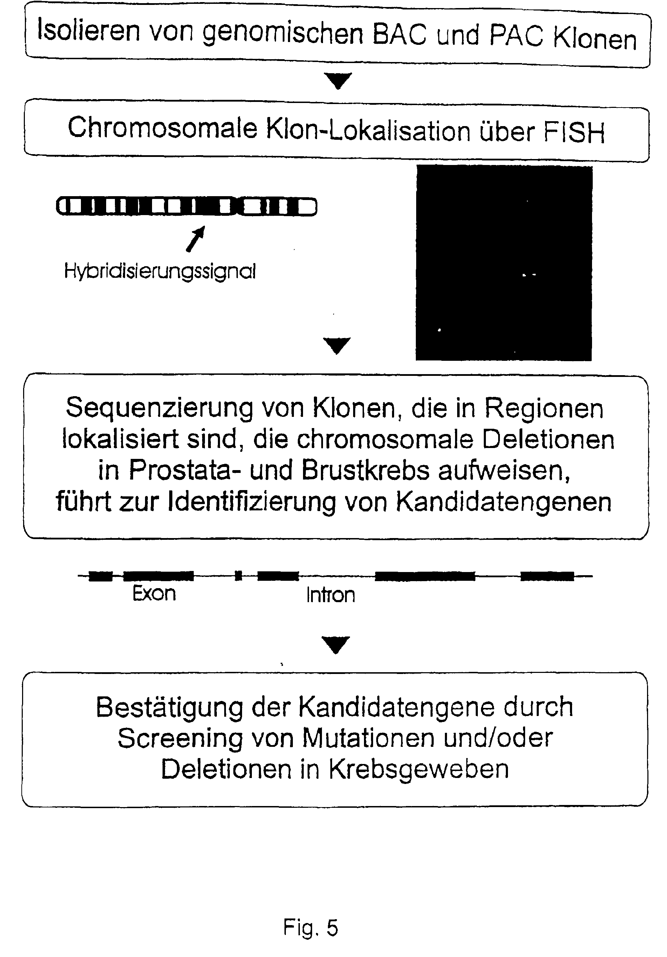

Mit den erfindungsgemäßen Nukleinsäuren (cDNA-Sequenzen) werden genomische BAC-, PAC- und Cosmid-Bibliotheken gescreent und über komplementäre Basenpaarung (Hybridisierung) spezifisch humane Klone isoliert. Die so isolierten BAC-, PAC- und Cosmid-Klone werden mit Hilfe der Fluoreszenz-in-situ-Hybridisation auf Metaphasenchromosomen hybridisiert und entsprechende Chromosomenabschnitte identifiziert, auf denen die entsprechenden genomischen Gene liegen. BAC-, PAC- und Cosmid-Klone werden sequenziert, um die entsprechenden genomischen Gene in ihrer vollständigen Struktur (Promotoren, Enhancer, Silencer, Exons und Introns) aufzuklären. BAC-, PAC- und Cosmid-Klone können als eigenständige Moleküle für den Gentransfer eingesetzt werden (s, Fig. 5).

Die Erfindung betrifft auch BAC-, PAC- und Cosmid-Klone, enthaltend funktionelle Gene und ihre chromosomale Lokalisation, entsprechend den Sequenzen Seq. ID No 1-123,258-273, zur Verwendung als Vehikel zum Gentransfer.

### Bedeutungen von Fachbegriffen und Abkürzungen

- Nukleinsäuren =: Unter Nukleinsäuren sind in der voliegenden Erfindung zu verstehen: mRNA, partielle cDNA, vollängen cDNA und genomische Gene (Chromosomen).
- ORF =: Open Reading Frame, eine definierte Abfolge von Aminosäuren, die von der cDNA-Sequenz abgeleitet werden kann.
- Contig =: eine Menge von DNA-Sequenzen, die aufgrund sehr großer Ähnlichkeiten zu einer Sequenz zusammengefaßt werden können (Consensus)
- Singleton =: ein Contig, der nur eine Sequenz enthält
- Modul =: Domäne eines Proteins mit einer definierten Sequenz, die eine strukturelle Einheit darstellt und in unterschiedlichen Proteinen vorkommt
- N =: wahlweise das Nukleotid A, T, G oder C
- X =: wahlweise eine der 20 natürlich vorkommenden Aminosäuren

### Erklärung zu den Alignmentparametern

- minimal initial match =: minimaler anfänglicher Identitätsbereich
- maximum pads per read =: maximale Anzahl von Insertionen
- maximum percent mismatch =: maximale Abweichung in %

### Erklärung der Abbildungen

- Fig. 1: zeigt die systematische Gen-Suche in der Incyte LifeSeq Datenbank.
- Fig. 2a: zeigt das Prinzip der EST-Assemblierung
- Fig. 2b1-2b4: zeigt das gesamte Prinzip der EST-Assemblierung
- Fig. 3: zeigt die in silico Subtraktion der Genexpression in verschiedenen Geweben
- Fig. 4a über: zeigt die Bestimmung der gewebsspezifischen Expression elektronischen Northern.
- Fig. 4b: zeigt den elektronischen Northem
- Fig. 5: zeigt die Isolierung von genomischen BAC- und PAC-Klonen.

Die nachfolgenden Beispiele erläutern die Herstellung der erfindungsgemäßen Nukleinsäure-Sequenzen, ohne die Erfindung auf diese Beispiele und Nukleinsäure-Sequenzen zu beschränken.

### Beispiel 1

### Suche nach Tumor-bezogenen Kandidatengenen

Zuerst wurden sämtliche ESTs des entsprechenden Gewebes aus der LifeSeq-Datenbank (vom Oktober 1997) extrahiert. Diese wurden dann mittels des Programms GAP4 des Staden-Pakets mit den Parametern 0% mismatch, 8 pads per read und einem minimalen match von 20 assembliert. Die nicht in die GAP4-Datenbank aufgenommenen Sequenzen (Fails) wurden erst bei 1% mismatch und dann nochmals bei 2% mismatch mit der Datenbank assembliert. Aus den Contigs der Datenbank, die aus mehr als einer Sequenz bestanden, wurden Consensussequenzen errechnet. Die Singletons der Datenbank, die nur aus einer Sequenz bestanden, wurden mit den nicht in die GAP4-Datenbank aufgenommenen Sequenzen bei 2% mismatch erneut assembliert. Wiederum wurden für die Contigs die Consensussequenzen ermittelt. Alle übrigen ESTs wurden bei 4% mismatch erneut assembliert. Die Consensussequenzen wurden abermals extrahiert und mit den vorherigen Consensussequenzen sowie den Singletons und den nicht in die Datenbank aufgenommenen Sequenzen abschließend bei 4% mismatch assembliert. Die Consensussequenzen wurden gebildet und mit den Singletons und Fails als Ausgangsbasis für die Gewebsvergleiche verwendet. Durch diese Prozedur konnte sichergestellt werden, daß unter den verwendeten Parametern sämtliche Sequenzen von einander unabhängige Genbereiche darstellten.

Fig. 2b1-2b4 veranschaulicht die Verlängerung der Ovartumorgewebe ESTs.

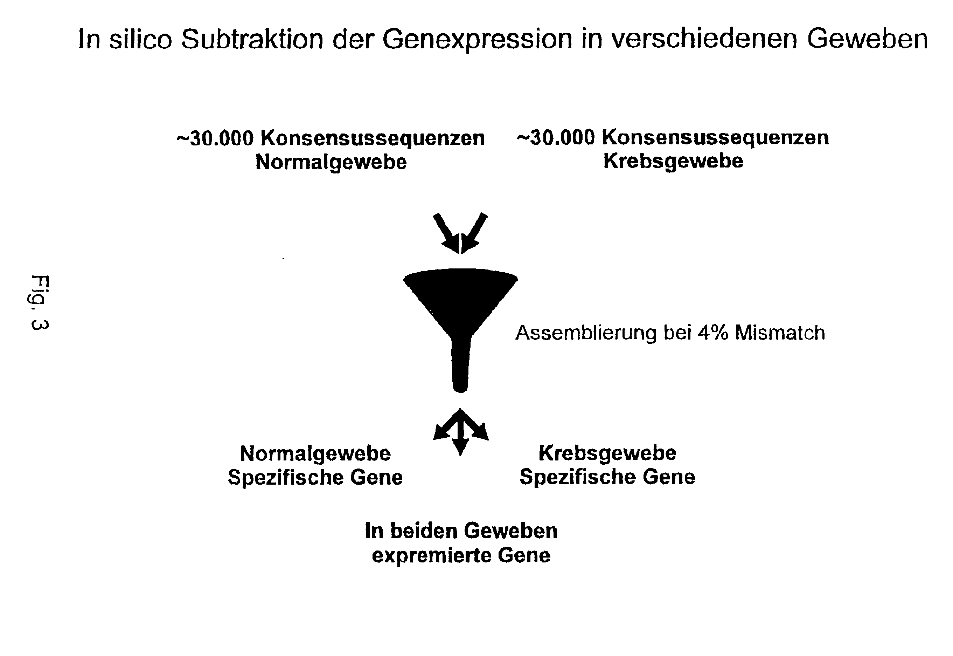

Die so assemblierten Sequenzen der jeweiligen Gewebe wurden anschließend mittels des gleichen Programms miteinander verglichen (Fig. 3). Hierzu wurden erst alle Sequenzen des ersten Gewebes in die Datenbank eingegeben. (Daher war es wichtig, daß diese voneinander unabhängig waren.)

Dann wurden alle Sequenzen des zweiten Gewebes mit allen des ersten verglichen. Das Ergebnis waren Sequenzen, die für das erste bzw. das zweite Gewebe spezifisch waren, sowie welche, die in beiden vorkamen. Bei Letzteren wurde das Verhältnis der Häufigkeit des Vorkommens in den jeweiligen Geweben ausgewertet. Sämtliche, die Auswertung der assemblierten Sequenzen betreffenden Programme, wurden selbst entwickelt

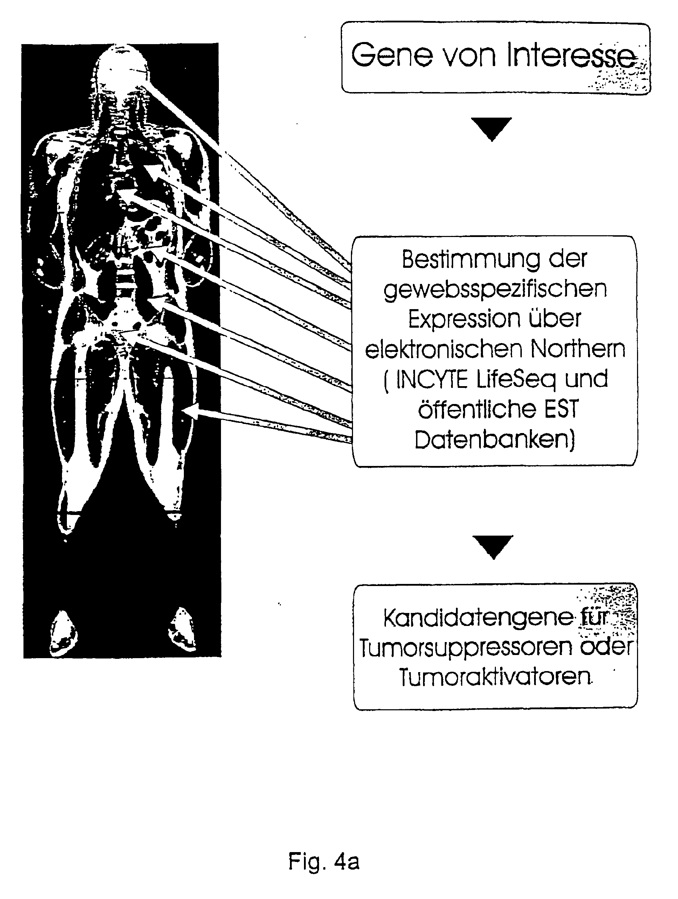

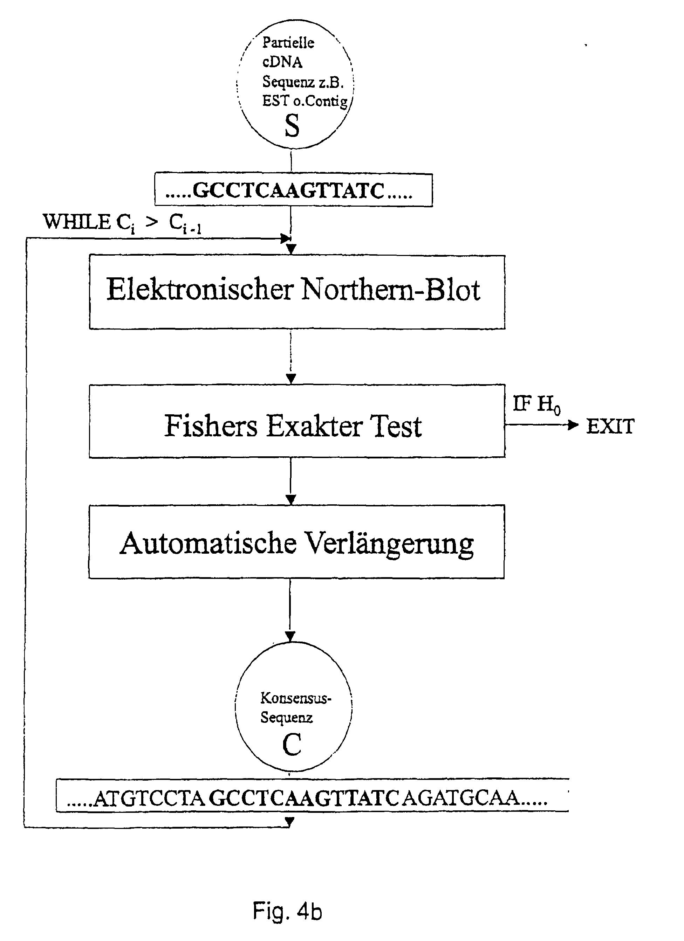

Alle Sequenzen, die mehr als viermal in jeweils einem der verglichenen Gewebe vorkamen, sowie alle, die mindestens fünfmal so häufig in einem der beiden Gewebe vorkamen wurden weiter untersucht. Diese Sequenzen wurden einem elektronischen Northern (s. Beispiel 2.1) unterzogen, wodurch die Verteilung in sämtlichen Tumor- und Normal-Geweben untersucht wurde (s. Fig. 4a und Fig. 4b). Die relevanten Kandidaten wurden dann mit Hilfe sämtlicher Incyte ESTs und allen ESTs öffentlicher Datenbanken verlängert (s. Beispiel 3). Anschließend wurden die Sequenzen und ihre Übersetzung in mögliche Proteine mit allen Nukleotid- und Proteindatenbanken verglichen, sowie auf mögliche, für Proteine kodierende Regionen untersucht.

### Beispiel 2

### Algorithmus zur Identifikation und Verlängerung von partiellen cDNA-Sequenzen mit verändertem Expressionsmuster

Im folgenden soll ein Algorithmus zur Auffindung über- oder unterexprimierter Gene erläutert werden. Die einzelnen Schritte sind der besseren Übersicht halber auch in einem Flußdiagramm zusammengefaßt (s. Fig. 4b).

### 2.1 Elektronischer Northern-Blot

Zu einer partiellen DNA-Sequenz *S*, z. B. einem einzelnen EST oder einem Contig von ESTs, werden mittels eines Standardprogramms zur Homolgiesuche, z. B. BLAST (Altschul, S. F., Gish W., Miller, W., Myers, E. W. und Lipman, D. J. (1990) *J. Mol. BioL*., **215,** 403-410), BLAST2 (Altschul, S. F., Madden, T. L., Schäffer, A. A., Zhang, J., Zhang, Z., Miller, W. und Lipman, D. J. (1997) *Nucleic Acids Research* **25** 3389-3402) oder FASTA (Pearson, W. R. und Lipman, D. J. (1988) Proc. Natl. Acad. Sci. USA **85** 2444-2448), die homologen Sequenzen in verschiedenen nach Geweben geordneten (privaten oder öffentlichen) EST-Bibliotheken bestimmt. Die dadurch ermittelten (relativen oder absoluten) Gewebe-spezifischen Vorkommenshäufigkeiten dieser Partial-Sequenz S werden als elektronischer Northern-Blot bezeichnet.

### 2.1.1

In analoger Verfahrensweise wurden auch der folgende Northern gefunden:

| Elektronischer Northern für SEQ. ID. NO: 115 | | | | |
|---|---|---|---|---|
| | NORMAL | TUMOR | Verhaeltnisse | |
| | %Haeufigkeit | %Haeufigkeit | N/T | T/N |
| Blase | 0.0039 | 0.0051 | 0.7627 | 1.3111 |
| Brust | 0.0000 | 0.0000 | undef | undef |
| Duenndarm | 0.0061 | 0.0000 | undef | 0.0000 |
| Eierstock | 0.0000 | 0.0702 | 0.0000 | undef |
| Endokrines_Gewebe | 0.0000 | 0.0000 | undef | undef |
| Gastrointestinal | 0.0192 | 0.0185 | 1.0354 | 0.9658 |
| Gehirn | 0.0007 | 0.0000 | undef | 0.0000 |
| Haematopoetisch | 0.0053 | 0.0000 | undef | 0.0000 |
| Haut | 0.0000 | 0.0000 | undef | undef |
| Hepatisch | 0.0000 | 0.0000 | undef | undef |
| Herz | 0.0000 | 0.0000 | undef | undef |
| Hoden | 0.0058 | 0.0000 | undef | 0.0000 |
| Lunge | 0.0052 | 0.0020 | 2.5402 | 0.3937 |
| Magen-Speigeroehre | 0.0193 | 0.0230 | 0.8404 | 1.1900 |
| Muskel-Skelett | 0.0000 | 0.0000 | undef | undef |
| Niere | 0.0000 | 0.0000 | undef | undef |
| Pankreas | 0.0017 | 0.0110 | 0.1496 | 6.6857 |
| Penis | 0.0000 | 0.0000 | undef | undef |
| Prostata | 0.0065 | 0.0000 | undef | 0.0000 |
| Uterus_Endometrium | 0.0000 | 0.0000 | undef | undef |
| Uterus_Myometrium | 0.0000 | 0.0000 | undef | undef |
| Uterus_allgemein | 0.0000 | 0.0954 | 0.0000 | undef |
| Brust-Hyperplasie | 0.0000 | | | |
| Prostata-Hyperplasie | 0.0000 | | | |
| Samenblase | 0.0000 | | | |
| Sinnesorgane | 0.0118 | | | |
| Welsse_Blutkoerperchen | 0.0000 | | | |
| Zervix | 0.0000 | | | |
| | FOETUS | | | |
| | %Haeufigkeit | | | |
| Entwicklung | 0.0000 | | | |
| Gastrointenstinal | 0.0028 | | | |
| Gehirn | 0.0000 | | | |
| Haematopoetisch | 0.0000 | | | |
| Haut | 0.0000 | | | |
| Hepatisch | 0.0000 | | | |
| Herz-Blutgefaesse | 0.0000 | | | |
| Lunge | 0.0000 | | | |
| Nebenniere | 0.0000 | | | |
| Niere | 0.0000 | | | |
| Placenta | 0.0000 | | | |
| Prostata | 0.0000 | | | |
| Sinnesorgane | 0.0000 | | | |
| | NORMIERTE/SUBTRAHIERTE BIBLIOTHEKEN | | | |
| | %Haeufigkeit | | | |
| Brust | 0.0000 | | | |
| Eierstock_n | 0.0000 | | | |
| Eierstock_ | t 0.0101 | | | |
| Endokrines_Gewebe | 0.0000 | | | |
| Foetal | 0.0047 | | | |
| Gastrointestinal | 0.0122 | | | |
| Haematopoetisch | 0.0114 | | | |
| Haut-Muskel | 0.0065 | | | |
| Hoden | 0.0000 | | | |
| Lunge | 0.0000 | | | |
| Nerven | 0.0010 | | | |
| Prostata | 0.0068 | | | |
| Sinnesorgane | 0.0000 | | | |
| Uterus_n | 0.0167 | | | |

### 2.2 Fisher-Test

Um zu entscheiden, ob eine Partial-Sequenz S eines Gens in einer Bibliothek für Normal-Gewebe signifikant häufiger oder seltener vorkommt als in einer Bibliothek für entartetes Gewebe, wird Fishers Exakter Test, ein statistisches Standardverfahren (Hays, W. L., (1991) Statistics, Harcourt Brace College Publishers, Fort Worth), durchgeführt.

Die Null-Hypothese lautet: die beiden Bibliotheken können bezüglich der Häufigkeit zu S homologer Sequenzen nicht unterschieden werden. Falls die Null-Hypothese mit hinreichend hoher Sicherheit abgelehnt werden kann, wird das zu S gehörende Gen als interessanter Kandidat für ein Krebs-Gen akzeptiert, und es wird im nächsten Schritt versucht, eine Verlängerung seiner Sequenz zu erreichen.

### Beispiel 3

### Automatische Verlängerung der Partial-Sequenz

Die automatische Verlängerung der Partial-Sequenz S vollzieht sich in drei Schritten:
1. Ermittlung aller zu *S* homologen Sequenzen aus der Gesamtmenge der zur Verfügung stehenden Sequenzen mit Hilfe von BLAST
2. Assemblierung dieser Sequenzen mittels des Standardprogramms GAP4 (Bonfield, J. K., Smith, K. F., und Staden R. (1995), Nucleic Acids Research **23** 4992-4999) (Contig-Bildung).
3. Berechnung einer Konsens-Sequenz *C* aus den assemblierten Sequenzen

Die Konsens-Sequenz *C* wird im allgemeinen länger sein als die Ausgangssequenz S. Ihr elektronischer Northern-Blot wird demzufolge von dem für *S* abweichen. Ein erneuter Fisher-Test entscheidet, ob die Alternativ-Hypothese der Abweichung von einer gleichmäßigen Expression in beiden Bibliotheken aufrechterhalten werden kann. Ist dies der Fall, wird versucht, *C* in gleicher Weise wie *S* zu verlängern. Diese Iteration wird mit der jeweils erhaltenen Konsensus-Sequenzen *C*_{*i*} (*i*: Index der Iteration) fortgesetzt, bis die Alternativ-Hypothese verworfen wird (if H₀ Exit; Abbruchkriterium I) oder bis keine automatische Verlängerung mehr möglich Ist (while *C*_{*i*} *> C*_{*i-1*}; Abbruchkriterium II).

Im Fall des Abbruchkriteriums II bekommt man mit der nach der letzten Iteration vorliegenden Konsens-Sequenz eine komplette oder annähernd komplette Sequenz eines Gens, das mit hoher statistischer Sicherheit mit Krebs in Zusammenhang gebracht werden kann.

Analog der oben beschriebenen Beispiele konnten die in der Tabelle I beschriebenen Nukleinsäure-Sequenzen aus Ovartumorgewebe gefunden werden.

Ferner konnten zu den einzelnen Nukleinsäure-Sequenzen die Peptidsequenzen (ORF's) bestimmt werden, die in der Tabelle II aufgelistet sind, wobei wenigen Nukleinsäure-Sequenzen kein Peptid zugeordnet werden kann und einigen Nukleinsäure-Sequenzen mehr als ein Peptid zugeordnet werden kann. Wie bereits oben erwähnt, sind sowohl die ermittelten Nukleinsäure-Sequenzen, als auch die den Nukleinsäure-Sequenzen zugeordneten Peptid-Sequenzen Gegenstand der vorliegenden Erfindung.

### Beispiel 4

### Kartierung der Nukleinsäure-Sequenzen auf dem humanen Genom

Die Kartierung der humanen Gene erfolgte unter Verwendung des Stanford G3 Hybrid-Panels (Stewart et al., 1997), der von Research Genetics, Huntsville, Alabama vertrieben wird. Dieses Panel besteht aus 83 verschiedenen genomischen DNAs von Mensch-Hamster Hybridzellinien und erlaubt eine Auflösung von 500 Kilobasen. Die Hybridzellinien wurden durch Fusion von bestrahlten diploiden menschlichen Zellen mit Zellen des Chinesischen Hamsters gewonnen. Das Rückhaltemuster der humanen Chromosomenfragmente wird mittels genspezifischer Primer in einer Polymerase-Kettenreaktion bestimmt und mit Hilfe der vom Stanford RH Server verfügbaren Software analysiert (http://www.stanford.edu/RH/rhserver_ form2.html). Dieses Programm bestimmt den STS-Marker, der am nächsten zum gesuchten Gen liegt. Die entsprechende zytogenetische Bande wurde unter Verwendung des "Mapview" -Programms der Genome Database (GDB), (http://gdbwww.dkfz-heidelberg.de) bestimmt. Neben dem kartieren von Genen auf dem menschlichen Cromosomensatz durch verschiedene experimentelle Methoden ist es möglich die Lage von Genen auf diesem durch bioinformatische Methoden zu bestimmen. Dazu wurde das bekannte Programm e-PCR eingesetzt (Schuler GD (1998) Electronic PCR: bridging the gap between genome mapping and genome sequencing. Trends Biotechnol 16; 456-459, Schuler GD (1997). Sequence mapping by electronic PCR. Genome Res 7; 541-550). Die dabei eingesetzte Datenbank entspricht nicht mehr der in der Literatur angegebenen, sonder ist eine Weiterentwicklung, welche Daten der öffentlichen Datenbank RHdb (http://www.ebi.ac.uk/RHdb/index.html) einschließt. Analog zu der Kartierung durch die Hybrid-Panels erfolgte eine Auswertung der Ergebnisse mit der obengenannten Software und der Software des Whitehead-Institutes (http://carbon.wi.mit.edu:8000/cgibin/contig/rhmapper.pl).

### Beispiel 5

### Gewinnung von genomischen DNA-Sequenzen (BAC-Klone)

Die die entsprechenden cDNA enthaltenen genomischen BAC-Klone (http://www.tree.caltech.edu/; Shizuya, H., B. Birren, U-J. Kim, V. Mancino, T. Slepak, Y. Tachiiri, M. Simon (1992) Proc. Natl. Acad. Sci., USA 89: 8794-8797) wurden mit der Prozedur des "down-to-the-well" isoliert. Bei dieser Prozedur wird eine Bibliothek bestehend aus BAC-Klonen (die Bibliothek überdeckt ca. 3 x das humane Genom) in ein bestimmtes Raster gebracht, so daß die DNA dieser Klone mit einer spezifischen PCR untersucht werden kann. Dabei erfolgt ein "Poolen" der DNA verschiedener BAC-Klone. Durch eine kombinatorische Analyse ist es möglich die Klone zu bestimmen, die die gesuchte DNA enthalten. Durch das Festlegen der Klone kann die Adresse der Klone in der Bibliothek bestimmt werden. Diese Adresse zusammen mit dem Namen der verwendeten Bibliothek legen die Klone und damit die DNA-Sequenz dieser Klone eindeutig fest. Die verwendete Bibliotheken waren CITB B und CITB C.

**TABELLE I**

| Seq ID No. | Expression | Funktion | Module | Cytogenetisch e Lokalisation |
|---|---|---|---|---|
| 115 | in Ovartumorgewebe überexprimiert | H.sapiens for neutrophil gelatinase assoclated lipocalin | lipocalin | 9q34 |

Die erfinderische Nukleinsäure-Sequenz Seq. ID No 115 des ermittelten Kandidatengenes wird in dem nachfolgenden Sequenzprotokoll beschrieben.

### Sequenzprotokoll

### (1) ALLGEMEINE INFORMATION:

(i) ANMELDER:
   (A) NAME: metaGen Pharmaceuticals GmbH
   (B) STRASSE: Oudenarder Str, 16
   (C) STADT: Berlin
   (E) LAND: Deutschland
   (F) POST CODE (ZIP): D-13347
   (G) TELEFON:
   (H) TELEFAX:
(ii) TITEL DER ERFINDUNG: Menschliche Nukleinsäure-Sequenzen aus Ovartumorgewebe
(iii) Anzahl der Sequenzen: 1

### 2) INFORMATION ÜBER SEQ ID NO: 115:

(i) SEQUENZ CHARAKTERISTIK:
   (A) LÄNGE: 719 Basenpaare
   (B) TYP: Nukleinsäure
   (C) STRANG: einzel
   (D) TOPOLOGIE: linear
(ii) MOLEKÜLTYP: aus einzelnen ESTs durch Assemblierung und Editierung hergestellte partielle cDNA
(iii) HYPOTHETISCH: NEIN
(iii) ANTI-SENSE: NEIN
(vi) HERKUNFT:
   (A) ORGANISMUS: MENSCH
   (C) ORGAN:
(vii) SONSTIGE HERKUNFT:
   (A) BIBLIOTHEK: cDNA library
(xi) SEQUENZ-BESCHREIBUNG: SEQ ID NO: 115:

gtcgactttt tttttttttt tttaacatgg aaaagtattt ttaaaaatcg aataatccta 60
ttcaagtcaa ccagtgttaa ccccggtgtg cttcctgcca gtctgttcct ccccatggga120
gtcacacaaa atgaaaatct cctagaaaga gaagacaaag acccgcaaaa gatgtatgcc180
accatctatg agctgaaaga agacaagagc tacaatgtca cctccgtcct gtttaggaaa240
aagaagtgtg actactggat caggactttt gttccaggtt gccagcccgg cgagttcacg300
ctgggcaaca ttaagagtta ccctggatta acgagttacc tcgtccgagt ggtgagcacc360
aactacaacc agcatgctat ggtgttcttc aagaaagttt ctcaaaacag ggagtacttc420
aagatcaccc tctacgggag aaccaaggag ctgacttcgg aactaaagga gaacttcatc480
cgcttctcca aatctctggg cctccctgaa aaccacatcg tcttccctgt cccaatcgac540
cagtgtatcg acggctgagt gcacaggtgc cgccagctgc cgcaccagcc cgaacaccat600
tgagggagct gggagaccct ccccacagtg ccacccatgc agctgctccc caggccaccc660
cgctgatgga gccccacctt gtctgctaaa taaacatgtg ccctcaaaaa aaaaaaaaa 719

## Patentansprüche

1. Eine Nukleinsäure-Sequenz, die ein Genprodukt oder ein Teil davon kodiert, umfassend
a) eine Nukleinsäure-Sequenz Seq. ID No 115,
b) eine allelische Variation der unter a) genannten Nukleinsäure-Sequenz
oder
c) eine Nukleinsäure-Sequenz, die komplementär zu den unter a) oder b) genannten Nukleinsäure-Sequenzen ist.

2. Eine Nukteinsäure-Sequenz gemäß Seq. ID No 115 oder eine komplementäre oder allelische Variante davon.

3. Nukleinsäure-Sequenz Seq. ID No 115, **dadurch gekennzeichnet, daß** sie in Ovartumorgewebe erhöht exprimiert ist.

6. Eine Nukleinsäure-Sequenz gemäß den Ansprüchen 1 bis 4, **dadurch gekennzeichnet, daß** sie eine 90% ige Homologie zu einer humanen Nukleinsäure-Sequenz aufweist,

7. Eine Nukleinsäure-Sequenz gemäß den Ansprüchen 1 bis 4, **dadurch gekennzeichnet, daß** sie eine 95% ige Homologie zu einer humanen Nukleinsäure-Sequenz aufweist.

8. Eine Nukleinsäure-Sequenz, umfassend einen Teil der in den Ansprüchen 1 bis 6 genannten Nukleinsäure-Sequenzen, in solch einer ausreichenden Größe, daß sie mit den Sequenzen gemäß den Ansprüchen 1 bis 7 hybridisieren.

9. Ein Nukleinsäure-Sequenz gemäß den Ansprüchen 1 bis 7, **dadurch gekennzeichnet, daß** die Größe des Fragments eine Länge von mindestens 50 bis 4500 bp aufweist.

10. Eine Nukleinsäure-Sequenz gemäß den Ansprüchen 1 bis 7, **dadurch gekennzeichnet, daß** die Größe des Fragments eine Länge von mindestens 50 bis 4000 bp aufweist.

11. Eine Nukleinsäure-Sequenz gemäß einem der Ansprüche 1 bis 10, die mindestens eine Teilsequenz eines biologisch aktiven Polypeptids kodiert.

12. , Eine Expressionskassette, umfassend ein Nukleinsäure-Fragment oder eine Sequenz gemäß einem der Ansprüche 1 bis 10, zusammen mit mindestens einer Kontroll- oder regulatorischen Sequenz.

13. Eine Expressionskassette, umfassend ein Nukleinsäure-Fragment oder eine Sequenz gemäß Anspruch 11, worin die Kontroll- oder regulatorische Sequenz ein geigneter Promotor ist.

14. Eine Expressionskassette gemäß einem der Ansprüche 12 und 13, **dadurch gekennzeichnet, daß** die auf der Kassette befindlichen DNA-Sequenzen ein Fusionsprotein kodieren, das ein bekanntes Protein und ein biologisch aktives Polypeptid-Fragment umfaßt.

15. Verwendung der Nukleinsäure-Sequenzen gemäß den Ansprüchen 1 bis 10 zur Herstellung von Vollängen-Genen.

16. Ein DNA-Fragment, umfassend ein Gen, das aus der Verwendung gemäß Anspruch 15 erhältlich ist.

17. Wirtszelle, enthaltend als heterologen Teil ihrer exprimierbaren genetischen Information ein Nukleinsäure-Fragment gemäß einem der Ansprüche 1 bis 10.

18. Wirtszelle gemäß Anspruch 17, **dadurch gekennzeichnet, daß** es ein prokaryontisches oder eukaryontische Zellsystem ist.

19. Wirtszelle gemäß einem der Ansprüche 17 oder 18, **dadurch gekennzeichnet, daß** das prokaryontische Zellsystem E. coli und das eukaryontische Zellsystem ein tierisches, humanes oder Hefe-Zellsystem ist.

20. Ein Verfahren zur Herstellung eines Polypeptids oder eines Fragments, **dadurch gekennzeichnet, daß** die Wirtszellen gemäß den Ansprüchen 17 bis 19 kultiviert werden.

21. Ein Antikörper, der gegen ein Polypeptid oder ein Fragment gerichtet ist, welches von der Nukleinsäure der Sequenz Seq. ID No 115 kodiert wird, das gemäß Anspruch 20 erhältlich ist.

22. Ein Antikörper gemäß Anspruch 20, **dadurch gekennzeichnet, daß** er monoklonal ist.

23. Ein Antikörper gemäß Anspruch 20 **dadurch gekennzeichnet, daß** er ein Phage-Display-Antikörper ist.

29. Verwendung der Nukleinsäure-Sequenzen gemäß Anspruch 3 in einem Phage Display Verfahren.

31. Verwendung der Nukleinsäure-Sequenzen gemäß den Sequenzen Seq. ID No 115 zur Expression von Polypeptiden, die als Tools zum Auffinden von Wirkstoffen gegen das Ovarialkarzinom verwendet werden können.

32. Verwendung der Nukleinsäure-Sequenzen Seq. ID No 115 in sense oder antisense Form.

36. Eine Nukleinsäure-Sequenz gemäß den Ansprüchen 1 bis 10, **dadurch gekennzeichnet, daß** es eine genomische Sequenz ist.

37. Eine Nukleinsäure-Sequenz gemäß den Ansprüchen 1 bis 10, **dadurch gekennzeichnet, daß** es eine mRNA-Sequenz ist.

38. Genomische Gene, ihre Promotoren, Enhancer, Silencer, Exonstruktur, Intronstruktur und deren Spleißvarianten, erhältlich aus der cDNA der Sequenz Seq. ID No 115.

39. Verwendung der genomischen Gene gemäß Anspruch 36, zusammen mit geeigneten regulativen Elementen.

40. Verwendung gemäß Anspruch 39, **dadurch gekennzeichnet, daß** das regulative Element ein geeigneter Promotor und/ oder Enhancer ist.

41. Eine Nukleinsäure-Sequenz gemäß den Ansprüchen 1 bis 7, **dadurch gekennzeichnet, daß** die Größe des Fragments eine Länge von mindestens 300 bis 3500 bp aufweist.
